# EUROPEAN PATENT APPLICATION

(11) **EP 4 588 426 A1**
(43) Date of publication of application: **23.07.2025**
(21) Application number: 23865282.0
(22) Date of filing: 30.08.2023
(51) Int. Cl.: A61B 3/16

(54) **PROBE FOR INTRAOCULAR PRESSURE MEASUREMENT AND INTRAOCULAR PRESSURE METER**

(30) Priority: 16.09.2022 JP 2022147809
(71) Applicant: INAMI & CO., LTD., Bunkyo-ku Tokyo 113-0033 (JP)
(72) Inventor: INAMI Takayuki, Tokyo 113-0033 (JP); NAITO Norihito, Tokyo 113-0033 (JP)
(74) Representative: Wächtershäuser & Hartz Patentanwaltspartnerschaft mbB
(86) International application number: PCT/JP2023/031371
(87) International publication number: WO 2024/057926

(57) **Abstract**

There is provided an instrument capable of measuring intraocular pressure of a subject with low invasiveness at higher accuracy. The instrument is a probe for intraocular pressure measurement 10 including a fiber optic pressure sensor 30 having a pressure receiver 38 provided at the distal end of an optical fiber 37 and a needle tube 20 that covers the pressure receiver 38 side of the fiber optic pressure sensor 30 and accommodates the pressure receiver 38 inside. The needle tube 20 has a needle tip 21 to be punctured into a subject at the distal end thereof and has a gauge size of 30 to 34 G. The pressure receiver 38 is provided at a position of the needle tip 21 inside the needle tube 20**.** The fiber optic pressure sensor 30 and the needle tube 20 are fixed on the proximal end 23 side of the needle tube 20 by a joint between the optical fiber 37 and the needle tube 20**.**

## Description

### Technical Field

The present invention relates to a probe for intraocular pressure measurement and a tonometer.

### Background Art

In the departments of ophthalmology of many hospitals, doctors have traditionally measured the intraocular pressure of a subject in order to diagnose eye diseases, such as, for example, glaucoma, and other diseases, and tonometers are used for the measurement. Although there have been highly invasive tonometers in the past, applanation tonometers have been mainly used in recent years because they can measure the intraocular pressure with low invasiveness.

The applanation tonometers include: non-contact tonometers, which do not directly contact the eye during the measurement; and contact tonometers, such as a Goldmann applanation tonometer, which directly contact the eye during measurement. In a non-contact tonometer, the intraocular pressure is measured by blowing an air stream onto the cornea of the eye of a subject (see, for example, Patent Literature 1). In a Goldmann applanation tonometer, the intraocular pressure is measured by bringing a prism into contact with the cornea of the eye of a subject (see, for example, Patent Literature 2). The Goldmann applanation tonometer can measure the intraocular pressure more accurately than the non-contact tonometers using air and therefore is used as a standard and is also used for the follow-up of glaucoma.

### Citation List

### Patent Literature

Patent Literature 1: Japanese Patent Publication No. 59-6652
Patent Literature 2: Japanese Patent Laid-Open No. 2001-128941

### Summary of Invention

### Technical Problem

The applanation tonometers described above perform measurement through the corneal tissue of the eye of a subject in both cases of the non-contact type using air and the contact type using a prism. Therefore, the measurement result depends on the corneal shape of the eye of the subject, and for example, a significant difference may occur in the intraocular pressure measurement values in an eye subjected to LASIK surgery, an eye subjected to corneal graft surgery, or the like.

In addition, as in the case of normal-tension glaucoma, which has been regarded as a problem in recent years, there are many patients who develop glaucoma due to damage to the optic nerve even though the intraocular pressure measured with an applanation tonometer gives a measured value within a normal range.

Accordingly, the present invention intends to provide an instrument capable of measuring intraocular pressure of a subject with low invasiveness at higher accuracy. The present invention also intends to provide a tonometer using the instrument.

### Solution to Problem

The present invention provides a probe for intraocular pressure measurement, including: a fiber optic pressure sensor having a pressure receiver provided at the distal end of an optical fiber; and a needle tube that covers the pressure receiver side of the fiber optic pressure sensor and accommodates the pressure receiver inside, wherein the needle tube has a needle tip to be punctured into a subject at the distal end thereof and has a gauge size of 30 to 34 G, the pressure receiver is provided at a position of the needle tip inside the needle tube, and the fiber optic pressure sensor and the needle tube are fixed on the proximal end side of the needle tube by a joint between the optical fiber and the needle tube.

The present invention also provides a tonometer including the probe for intraocular pressure measurement.

### Advantageous Effects of Invention

The present invention makes it possible to provide a probe for intraocular pressure measurement as an instrument capable of measuring intraocular pressure of a subject with low invasiveness at higher accuracy. The present invention also makes it possible to provide a tonometer using the probe for intraocular pressure measurement.

### Brief Description of Drawings

[Figure 1] Figure 1 is an appearance drawing illustrating an example of a probe for intraocular pressure measurement of one embodiment of the present invention.
[Figure 2] Figure 2 is an enlarged view of region A in Figure 1.
[Figure 3] Figure 3 is a cross-sectional view cut along line B-B in Figure 2.
[Figure 4] Figure 4 is an enlarged view of region C in Figure 3.
[Figure 5] Figure 5 is an enlarged view of region D in Figure 3.
[Figure 6] Figure 6 is a schematic configuration diagram of a pressure receiver of a fiber optic Fabry-Perot pressure sensor.
[Figure 7] Figure 7 is a schematic configuration diagram of a tonometer of one embodiment of the present invention.
[Figure 8] Figure 8 is a cross-sectional view of an eyeball, illustrating the structure of an eyeball.

### Description of Embodiments

In the case of applanation tonometers, such as non-contact tonometers using air and contact tonometers using a prism (Goldmann applanation tonometers), measurement is performed through the corneal tissue of the subject's eye, so the measurement results depend on the corneal shape. Therefore, there is a first problem that the intraocular pressure measurement values by the applanation tonometers vary depending on, for example, the histological change of the cornea due to LASIK surgery or the like, or the condition of the subject, making it difficult to measure the intraocular pressure as an absolute value.

On the other hand, glaucoma is originally a disease in which the optic nerve is damaged due to an increase in intraocular pressure and the visual field is narrowed or partially lost, but even a person whose intraocular pressure is within a normal range may develop glaucoma, and this is called normal-tension glaucoma. In recent years, normal-tension glaucoma accounts for about 70% of cases of glaucoma, and there is a second problem that cases of developing glaucoma due to damage to the optic nerve are increasing even though the intraocular pressure measured by an applanation tonometer is within a normal range.

Based on the above-described first and second problems, the present inventors have hypothesized that the intraocular pressure may be different between the cornea side (anterior chamber side) and the retina side of the eyeball of a subject and verified the hypothesis by animal experiments using a micro pressure sensor. As a result, it has been found that there is a significant difference in the value of intraocular pressure between the cornea side and retina side of the rabbit, and thus the present inventors have studied a probe for intraocular pressure measurement as a medical instrument and have completed the present invention.

Specifically, a probe for intraocular pressure measurement of one embodiment of the present invention includes: a fiber optic pressure sensor having a pressure receiver provided at the distal end of an optical fiber; and a needle tube that covers the pressure receiver side of the fiber optic pressure sensor and accommodates the pressure receiver inside. The needle tube has a needle tip to be punctured into a subject at the distal end thereof and has a gauge size of 30 to 34 G. In addition, in this probe for intraocular pressure measurement, the pressure receiver of the fiber optic pressure sensor is provided at a position of the needle tip inside the needle tube. Furthermore, the fiber optic pressure sensor and the needle tube are fixed on the proximal end side of the needle tube by a joint between the optical fiber and the needle tube.

In the probe for intraocular pressure measurement of the present embodiment, the needle tube having a needle tip to be punctured into a subject has a gauge size of 30 to 34G, and therefore the intraocular pressure can be measured with low invasiveness. In addition, in the probe for intraocular pressure measurement of the present embodiment, the pressure receiver of the fiber optic pressure sensor is provided at a position of the needle tip inside the needle tube, and the fiber optic pressure sensor and the needle tube are fixed on the proximal end side of the needle tube by a joint between the optical fiber and the needle tube, therefore the intraocular pressure can be measured with extremely high accuracy.

Hereinafter, preferred configurations, specific examples, and the like of a probe for intraocular pressure measurement of one embodiment of the present invention and a tonometer including the probe for intraocular pressure measurement will be described in detail with reference to the drawings, but the present invention is not limited to the following embodiment. Note that in the drawings, common components are denoted by the same reference numerals, and redundant description thereof may be omitted.

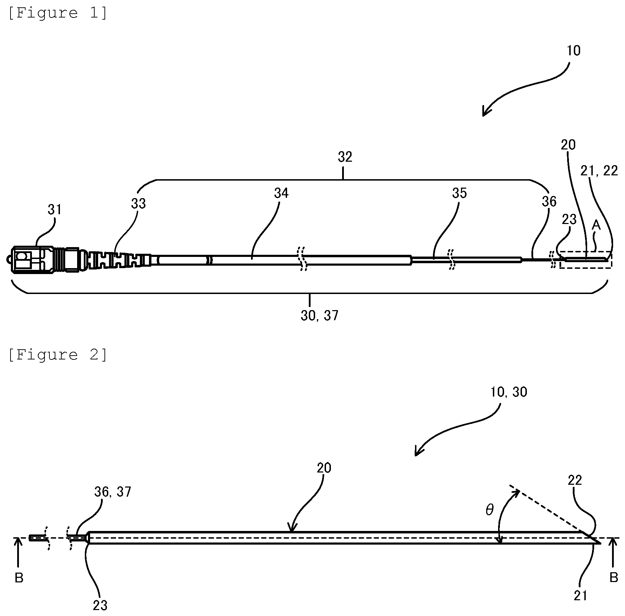

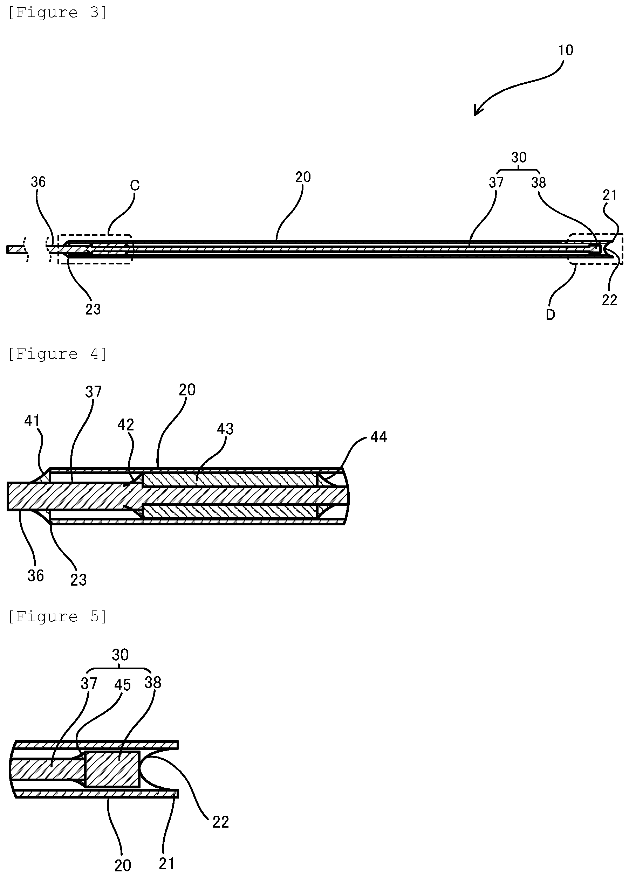

Figure 1 is an appearance drawing illustrating a probe for intraocular pressure measurement 10 as an example of one embodiment of the present invention. Figure 2 is an enlarged view of region A in Figure 1 and is a partially enlarged view of the distal end side of the probe for intraocular measurement 10 shown in Figure 1. Figure 3 is a cross-sectional view cut along line B-B in Figure 2 and is a cross-sectional view along the axis direction on the distal end side of the probe for intraocular pressure measurement 10 shown in Figure 1. Figure 4 is an enlarged view of region C in Figure 3. Figure 5 is an enlarged view of region D in Figure 3.

The probe for intraocular pressure measurement 10 includes: a needle tube 20 on the distal end side of the probe for intraocular pressure measurement 10; and a fiber optic pressure sensor 30 whose distal end side is covered with the needle tube 20. As shown in Figure 1, the probe for intraocular pressure measurement 10 can include: a connector 31 that is connected to an apparatus main body of a tonometer described later; and a covering part 32 that covers an optical fiber 37 connected to the connector 31. The covering part 32 may be composed of, for example, a cable 33, a cable jacket 34, a resin tube 35, and a resin coat 36.

As shown in Figure 2, the needle tube 20 on the distal end side of the probe for intraocular pressure measurement 10 has a needle tip 21 to be punctured into a subject at the distal end thereof. The needle tube 20 has a hollow cylindrical shape, covers the distal end side of the fiber optic pressure sensor 30 described later, and accommodates, inside the needle tube 20, a pressure receiver described later in the fiber optic pressure sensor 30. A blade surface 22 is formed at the needle tip 21 of the needle tube 20 so as to puncture the tissue of a subject, and the angle, θ, of the blade surface 22 is preferably 5 to 40°, more preferably 5 to 30°. In addition, the angle, θ, of the blade surface 22 is also preferably 20 to 40°, more preferably 25 to 35°.

The needle tube 20 has a gauge size of 30 to 34 G from the viewpoint of low invasiveness and covering the distal end side of the fiber optic pressure sensor 30. The gauge size of the needle tube 20 represents the outer diameter (thickness) of the needle tube 20, and the higher the gauge size is, the thinner the needle tube 20 is. For example, the outer diameter of the needle tube 20 made of SUS304 and having a gauge size of 28 to 34 G is about 0.36 to about 0.18 mm, and the inner diameter thereof is about 0.20 to about 0.10 mm; and the outer diameter of the needle tube 20 made of SUS304 and having a gauge size of 30 to 34 G is about 0.30 to about 0.18 mm, and the inner diameter thereof is about 0.14 to about 0.10 mm. The gauge size of the needle tube 20 is 30 G or higher from the viewpoint of low invasiveness. On the other hand, the gauge size of the needle tube 20 is preferably 33 G or lower, more preferably 32 G or lower from the viewpoint of covering the distal end side of the fiber optic pressure sensor 30.

When the needle tube 20 covers the distal end side of the fiber optic pressure sensor 30, thereby the rigidity is imparted to the probe for intraocular pressure measurement 10. This makes it easy for a user such as a doctor to handle the probe for intraocular pressure measurement 10. The length of the needle tube 20 is preferably 10 to 20 mm, more preferably 12.5 to 18.5 mm, from the viewpoint of operability for users.

The material of the needle tube 20 is preferably stainless steel, such as SUS304, SUS304L, SUS316, SUS321, SUS430, SUS201, and SUS202. Besides the stainless steel, the needle tube 20 made of another metal, such as, for example, titanium and titanium alloy, may be used.

As shown in Figure 3, the fiber optic pressure sensor 30 has an optical fiber 37 and a pressure receiver 38 provided at the distal end of the optical fiber 37. The outer diameter of the optical fiber 37, including the pressure receiver 38 provided at the distal end thereof, is smaller than the inner diameter of the needle tube 20 at least in the part accommodated inside the needle tube 20. The outer diameter of the optical fiber 37 is preferably 200 µm or less for example.

The distal end side of the fiber optic pressure sensor 30 is a part having a predetermined length from the distal end surface of the pressure receiver 38 in the part where the fiber optic pressure sensor 30 is covered with the needle tube 20. The distal end side of the optical fiber 37 is a part having a predetermined length from the distal end of the optical fiber 37 provided with the pressure receiver 38 in the part where the fiber optic pressure sensor 30 is covered with the needle tube 20. At the distal end surface of the optical fiber 37, the core and cladding of the optical fiber 37 are exposed. In addition, at the distal end side (the part covered with the needle tube 20) of the optical fiber 37, the cladding is preferably exposed without being covered with the covering part 32. On the other hand, the other part of the optical fiber 37, other than the part accommodated inside the needle tube 20, is preferably covered with the covering part 32 composed of, for example, the cable 33, the cable jacket 34, the resin tube 35, and the resin coat 36.

For example, the length from the distal end to the proximal end (connector 31 side) of the fiber optic pressure sensor 30 is preferably 2,500 to 3,100 mm, more preferably 2700 to 2900 mm**.** In addition, as shown in Figure 1, a part having a predetermined length from the proximal end side of the optical fiber 37 may be covered with the connector 31, a cable 33, the cable jacket 34, and the like. Furthermore, a part of the optical fiber 37, the part having a predetermined length from the part covered with the cable jacket 34, may be covered with the resin tube 35, made of a fluororesin or the like, and a part from the part covered with the resin tube 35 to the proximal end 23 of the needle tube 20 may be coated with the resin coat 36, such as polyimide. Note that the optical fiber 37 may be divided into a plurality of pieces in the length direction thereof and optically connected with connectors or the like.

As shown in Figure 3 and Figure 5, the distal end of the optical fiber 37 of the fiber optic pressure sensor 30 is provided with the pressure receiver 38. The pressure receiver 38 is composed of a diaphragm (such as a stainless steel diaphragm and a silicon diaphragm), and when the diaphragm receives pressure of a gas or liquid, thereby displacement, such as deformation, bending, and distortion, occurs. The fiber optic pressure sensor 30 optically measures the displacement or the like of the pressure receiver (diaphragm) due to pressure.

As described above, the pressure receiver 38 side of the fiber optic pressure sensor 30 is covered with the needle tube 20. The pressure receiver 38 is accommodated inside the needle tube 20 and is provided at a position of the needle tip 21 inside the needle tube 20. Therefore, when the needle tip 21 of the probe for intraocular pressure measurement 10 is punctured into the tissue (for example, the vitreous body) of the eye of a subject, thereby the intraocular pressure can be measured with extremely high accuracy with the pressure receiver 38 provided at the position of the needle tip 21 inside the needle tip 20.

The fiber optic pressure sensor 30 and the needle tube 20 are configured in such a way as to be fixed on the proximal end 23 side of the needle tube 20 by a joint between the optical fiber 37 and the needle tube 20. Regarding the configuration, as shown in Figure 4 for example, the fiber optic pressure sensor 30 and the needle tube 20 are preferably fixed on the proximal end 23 side of the needle tube 20 by joints 41, 42, 43, 44 by means of an adhesive, fused quartz, or welding between the optical fiber 37 of the fiber optic pressure sensor 30 and the needle tube 20. The positions and number of joints between the optical fiber 37 and the needle tube 20 are not limited to the configuration shown in the figure as long as at least one joint is present at a spot on the proximal end 23 side, and a plurality of joints may be present at a plurality of spots. In addition, as shown in Figure 4, when a plurality of joints is provided at a plurality of spots, the joints of the same material may be provided at the spots, or the joints of different materials may be provided depending on the respective spots (positions). Furthermore, as shown in Figure 5, the optical fiber 37 and the pressure receiver 38 of the fiber optic pressure sensor 30 are preferably fixed by a joint 45 by means of an adhesive, welding, or the like.

Suitable specific examples of the fiber optic pressure sensor 30 include a fiber optic Fabry-Perot pressure sensor. The fiber optic Fabry-Perot pressure sensor is a fiber optic pressure sensor 30 making use of the principle of a Fabry-Perot interferometer.

Here, the Fabry-Perot interferometer basically includes: two mirrors facing each other; and a space (Fabry-Perot cavity) between the mirrors. When the light reflected by the reflection surface of the near-side mirror and the light reflected by the reflection surface of the far-side mirror are superimposed on each other, interference occurs due to a phase difference caused by the optical path length difference. The reflected light intensity obtained by interference varies depending on the optical path length (cavity length × refractive index) between the two reflection surfaces. From this, a Fabry-Perot interferometer can be used as a sensor by making a structure in which the cavity length or the refractive index changes. The light reflected in the Fabry-Perot interferometer is precisely wavelength modulated according to the cavity length. In order to accurately measure the pressure or the like that affects the cavity length, it is necessary to measure the cavity length stably and accurately, and from that viewpoint, a white light cross-correlator is preferably used.

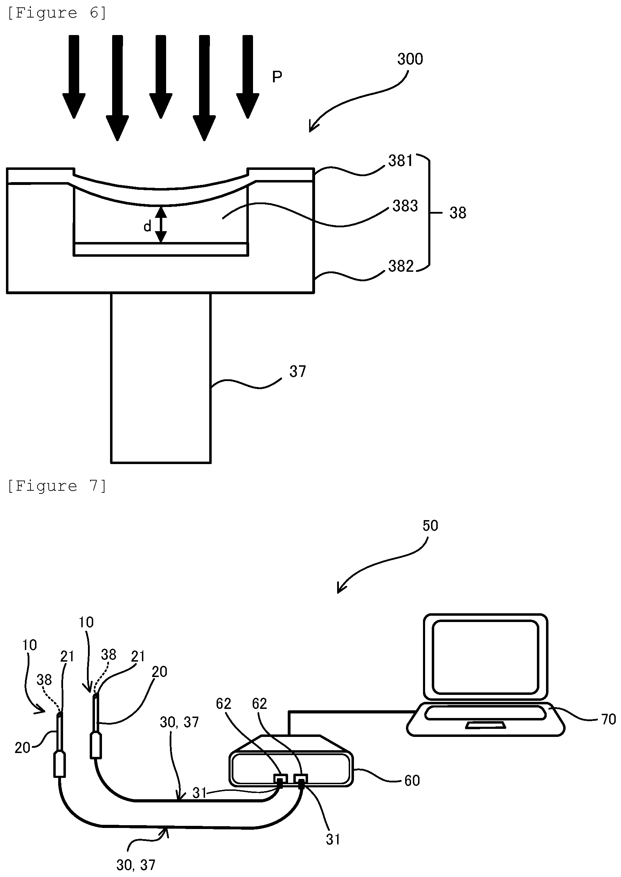

A fiber optic Fabry-Perot pressure sensor measures the displacement of a diaphragm due to pressure as a change in the distance between reflective surfaces. As shown in Figure 6, a more preferred fiber optic Fabry-Perot pressure sensor 300 includes: an optical fiber 37; and a micro-optical mechanical system (MOMS) as a pressure receiver 38 provided at the distal end of the optical fiber 37. The MOMS (pressure receiver 38) includes a deformable membrane 381 assembled over a vacuum cavity to form a micro-drum-like structure 382. The bottom of the drum-like structure 382 and the inner surface of the flexible membrane 381 form a Fabry-Perot cavity 383 with cavity length d, and when pressure P is applied, the membrane 381 bends towards the bottom of the drum-like structure 382, which shortens the cavity length d. As just described above, in the fiber optic Fabry-Perot pressure sensor 300 shown in Figure 6, distortion occurs according to the pressure change in the mirror section disposed at the distal end of the optical fiber 37, and the light polarization caused by the distortion is output as an accurate pressure change based on the Fabry-Perot interference.

As the fiber optic Fabry-Perot pressure sensor 300, commercially available ones can be used. Examples thereof include "OPP-M" series, trade name, manufactured by Opsens Inc., and "FOP-M" series such as "FOP-M200", trade name, manufactured by FISO Technologies, Inc.

Next, a tonometer of one embodiment of the present invention, the tonometer including the above-described probe for intraocular pressure measurement 10, will be described. Figure 7 is a schematic diagram illustrating a tonometer 50 as one example thereof. The tonometer 50 includes: the above-described probe for intraocular pressure measurement 10; and an apparatus main body 60. Note that in Figure 7, the probe for intraocular pressure measurement 10 is illustrated in a simplified manner.

In the tonometer 50, the probe for intraocular pressure measurement 10 is connected to the apparatus main body 60 through the connector 31 connected to the optical fiber 37. The apparatus main body 60 has a function of converting optical information on pressure (intraocular pressure) from the fiber optic pressure sensor 30 into electric signals and outputting the electric signals. As the apparatus main body 60, a signal conditioner for receiving and reading information and signals on pressure (intraocular pressure) from the pressure receiver 38 of the fiber optic pressure sensor 30 through the optical fiber 37 can be used. For example, when the fiber optic Fabry-Perot pressure sensor 300, which is more suitable as the fiber optic pressure sensor 30, is used, a signal conditioner for reading the cavity length d of the Fabry-Perot cavity 383 can be used suitably as the apparatus main body 60.

The apparatus main body 60, such as a signal conditioner as described above, is connected to the fiber optic pressure sensor 30 and therefore can include a built-in light source (light emitting apparatus), a built-in light-receptive apparatus, and the like, although the illustration is omitted. In addition, the apparatus main body 60 may include, for example, a control unit composed of CPU, a microprocessor, a microcomputer, and the like, and a memory unit that stores the information on subjects, the measurement data, and the like, or the apparatus main body 60 may be connected to a personal computer (PC such as desktop PC, note PC, and tablet PC) 70 including a control unit and a memory unit. The control unit has functions such as: controlling the behavior of the apparatus main body 60, such as light emission or light receiving; and performing computational processing for converting light signals from the fiber optic pressure sensor 30 to electric signals and converting the electric signals to pressure measurement values. For example, software (program) for recording and analyzing signals may be installed in the memory unit of the apparatus main body 60 and the PC 70, and the control unit may read out the software to execute, for example, reading out, converting, and outputting various signals. The software may include various correction functions such as automatic calibration of the fiber optic pressure sensor 30 and automatic identification.

Furthermore, although the illustration is omitted, the apparatus main body 60 may include: an input apparatus for a user such as a doctor to operate the apparatus main body 60; and a display apparatus for displaying the operation state of the apparatus main body 60, the pressure waveforms (such as, for example, a pulsed rectangular wave) obtained by the fiber optic pressure sensor 30, and the like. In such a display apparatus, it is also possible to monitor the pressure (intraocular pressure) in real time, and it is also possible to display an average value, a maximum value, a minimum value, and the like within a set measurement time.

The apparatus main body 60 can also include a plurality of connectors 62 for connecting the apparatus main body 60 to the probe for intraocular pressure measurement 10, and Figure 7 shows two connectors 62 and illustrates a configuration such that two probes for intraocular pressure measurement 10 are connected to the apparatus main body 60. In this way, by using a plurality of probes for intraocular pressure measurement 10, the intraocular pressure can be measured at a plurality of arbitrary spots in an eyeball. For example, by measuring the intraocular pressure on the vitreous body side with one channel and measuring the intraocular pressure on the cornea side (anterior chamber side) with the other channel, it is possible to perform a more precise examination while taking the influence of the eyeball tissue such as the cornea into consideration.

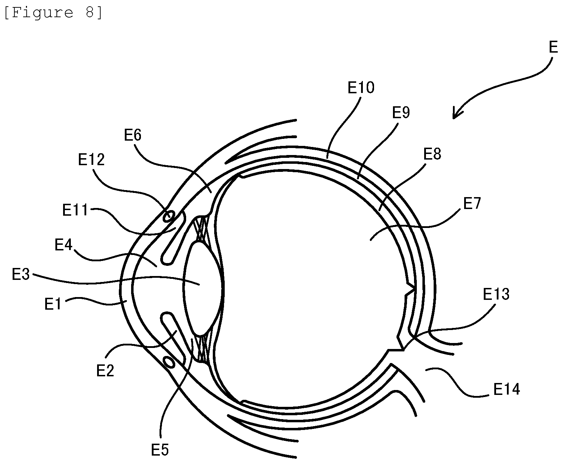

Next, a preferred mode of use of the above-described probe for intraocular pressure measurement 10 and tonometer 50 including the same will be described with reference to Figure 8. Figure 8 is a cross-sectional view of an eyeball for describing the structure of an eyeball E. As shown in Figure 8, the eyeball E has tissues such as a cornea E1, an iris E2, a crystalline lens E3, an anterior chamber E4, a posterior chamber E5, a ciliary body E6, a vitreous body E7, a retina E8, a choroid coat E9, a sclerotic coat E10, a chamber angle E11, a Schlemm's canal E12, an optic nerve head E13, and an optic nerve E14.

The probe for intraocular pressure measurement 10 makes it possible to measure the intraocular pressure directly by the fiber optic pressure sensor 30 including the pressure receiver 38 provided at the position of the needle tip 21 inside the needle tube 20 and therefore is preferably used in a mode such that the needle tip 21 is punctured into the vitreous body E7 of the eye of a subject. This makes it possible to measure the intraocular pressure at a position closer to the optic nerve head E13 and the optic nerve 14 as compared to when the intraocular pressure is measured from the cornea E1 side, so that the intraocular pressure can be measured with higher accuracy. This is because the pressure is considered to be the same value wherever it is measured within the posterior eye segment. Although the vitreous body E7 is a gel-like tissue, the fiber optic pressure sensor 30 can measure the pressure (intraocular pressure) even through the gel-like tissue.

As described above, the probe for intraocular pressure measurement 10 is used in a mode such that it is punctured into a tissue of the eyeball E of a subject and therefore is preferably disposed of each time it is used, that is, the probe for intraocular pressure measurement 10 is preferably a disposable article. Note that the probe for intraocular pressure measurement 10 may be handled by hand by a user such as a doctor or may be handled by robot control.

As described above in detail, in the probe for intraocular pressure measurement of the present embodiment, the needle tube which covers the pressure receiver side of the fiber optic pressure sensor to accommodate the pressure receiver inside thereof and has a gauge size of 30 to 34 G and the fiber optic pressure sensor are fixed on the proximal side of the needle tube by a joint between the optical fiber and the needle tube. Therefore, the pressure receiver of the fiber optic pressure sensor can be fixed and provided at the position of the needle tip inside the needle tube. Accordingly, when the probe for intraocular pressure measurement and tonometer including the same of the present embodiment is used and the needle tip of the probe for intraocular pressure measurement is punctured into a tissue of the eye of a subject, thereby the intraocular pressure of the subject can be measured with low invasiveness at higher accuracy due to the pressure receiver provided at the position of the needle tip inside the needle tube. The intraocular pressure can be measured at higher accuracy by using the tonometer including this probe for intraocular pressure measurement as compared to when conventional applanation tonometers are used, in addition, the conventional applanation tonometers can measure the intraocular pressure simply and therefore are suitable for screening and daily examinations, and accordingly the probe for intraocular pressure measurement and the tonometer are suitably used for a thorough intraocular pressure examination.

For example, when a measurement result such as a suspicion of glaucoma is obtained in intraocular pressure measurement with a conventional tonometer and such a measurement result is also obtained after a certain follow-up, the tonometer including the probe for intraocular pressure measurement of the present embodiment can be suitably used for a thorough intraocular pressure examination. In addition, for example, in intraocular pressure measurement with a conventional tonometer, when there is no abnormality in the measurement result but there is a symptom suspected of glaucoma, the tonometer including the probe for intraocular pressure measurement of the present embodiment can be suitably used for a thorough intraocular pressure examination.

Furthermore, ophthalmic surgery is always performed so as to keep a constant intraocular pressure while paying attention to fluctuations in the intraocular pressure, and therefore the tonometer including the probe for intraocular pressure measurement of the present embodiment can be used for monitoring the intraocular pressure during ophthalmic surgery. In addition, the use of the probe for intraocular pressure measurement and tonometer of the present embodiment has a possibility of creating a new treatment method for glaucoma for which there is currently no sure cure and there is only a treatment method of lowering the intraocular pressure, and therefore such medical contribution is also expected.

Note that the above-described one embodiment of the present invention can take the following configurations.
[1] A probe for intraocular pressure measurement, including:
   a fiber optic pressure sensor having a pressure receiver provided at the distal end of an optical fiber; and
   a needle tube that covers the pressure receiver side of the fiber optic pressure sensor and accommodates the pressure receiver inside, wherein
   the needle tube has a needle tip to be punctured into a subject at the distal end thereof and has a gauge size of 30 to 34 G,
   the pressure receiver is provided at a position of the needle tip inside the needle tube, and
   the fiber optic pressure sensor and the needle tube are fixed on the proximal end side of the needle tube by a joint between the optical fiber and the needle tube.
[2] The probe for intraocular pressure measurement according to [1], wherein the fiber optic pressure sensor is a fiber optic Fabry-Perot pressure sensor.
[3] The probe for intraocular pressure measurement according to [1] or [2], wherein an angle of a blade surface of the needle tip of the needle tube is 5 to 40°.
[4] The probe for intraocular pressure measurement according to any one of [1] to [3], wherein the probe for intraocular pressure measurement is used in a mode in which the needle tip is punctured into the vitreous body of the eye of the subject.
[5] A tonometer including the probe for intraocular pressure measurement according to any one of [1] to [4].

### Reference Signs List

- 10: Probe for intraocular pressure measurement
- 20: Needle tube
- 21: Needle tip
- 22: Blade surface
- 30: Fiber optic pressure sensor
- 37: Optical fiber
- 38: Pressure receiver
- 50: Tonometer
- 60: Apparatus main body

## Claims

1. A probe for intraocular pressure measurement, comprising:
a fiber optic pressure sensor having a pressure receiver provided at the distal end of an optical fiber; and
a needle tube that covers the pressure receiver side of the fiber optic pressure sensor and accommodates the pressure receiver inside, wherein
the needle tube has a needle tip to be punctured into a subject at the distal end thereof and has a gauge size of 30 to 34 G,
the pressure receiver is provided at a position of the needle tip inside the needle tube, and
the fiber optic pressure sensor and the needle tube are fixed on the proximal end side of the needle tube by a joint between the optical fiber and the needle tube.

2. The probe for intraocular pressure measurement according to claim 1, wherein the fiber optic pressure sensor is a fiber optic Fabry-Perot pressure sensor.

3. The probe for intraocular pressure measurement according to claim 1, wherein an angle of a blade surface of the needle tip of the needle tube is 5 to 40°.

4. The probe for intraocular pressure measurement according to claim 1, wherein the probe for intraocular pressure measurement is used in a mode in which the needle tip is punctured into the vitreous body of the eye of the subject.

5. A tonometer comprising the probe for intraocular pressure measurement according to any one of claims 1 to 4.
